# EUROPEAN PATENT SPECIFICATION

(11) **EP 2 536 433 B1**
(45) Date of publication and mention of the grant of the patent: **02.08.2017**
(21) Application number: 11745457.9
(22) Date of filing: 22.02.2011
(51) Int. Cl.: C07K 16/28, C12N 15/113, A61K 39/395, A61P 27/02, A61P 27/00, A61K 31/7105, A61K 38/16

(54) **EPITHELIAL MEMBRANE PROTEIN 2 (EMP2) BINDING REAGENTS AND THEIR THERAPEUTIC USES IN OCULAR DISEASES**
BINDEREAGENZIEN DES EPITHELIALMEMBRANENPROTEIN-2 (EMP2) UND DEREN THERAPEUTISCHEN VERWENDUNGEN BEI AUGENKRANKHEITEN
RÉACTIFS DE LIAISON DE LA PROTÉINE MEMBRANAIRE ÉPITHÉLIALE 2 (EMP2) ET LEURS UTILISATIONS THÉRAPEUTIQUES DANS DES MALADIES OCULAIRES

(30) Priority: 19.02.2010 US 306073 P
(43) Date of publication of application: 26.12.2012
(73) Proprietor: The Regents of the University of California, Oakland, CA 94607 (US); United States Government Department Of Veterans Affairs, Washington, DC 20420 (US)
(72) Inventor: GORDON, Lynn K., Los Angeles, CA 90095-1406 (US); BRAUN, Jonathan, Los Angeles, CA 90095-1406 (US); MORALES, Shawn, Los Angeles, CA 90095-1406 (US); TELANDER, David, G., Los Angeles, CA 90095-1406 (US); WADEHRA, Madhuri, Los Angeles, CA 90095-1406 (US)
(74) Representative: Harrison IP Ltd.
(86) International application number: PCT/US2011/025772
(87) International publication number: WO 2011/103599

(56) References cited:
- WO-A2-2009/048980
- WO-A2-2011/063161
- SHAWN A MORALES ET AL: "FAK activation and the role of epithelial membrane protein 2 (EMP2) in collagen gel contraction", INVESTIGATIVE OPHTHALMOLOGY & VISUAL SCIENCE, ASSOCIATION FOR RESEARCH IN VISION AND OPHTHALMOLOGY, US, vol. 50, no. 1, 1 January 2009 (2009-01-01), pages 462-469, XP008157989, ISSN: 0146-0404, DOI: 10.1167/IOVS.07-1598
- SORBERA L A ET AL: "RANIBIZUMAB: TREATMENT OF AGE-RELATED MACULAR DEGENERATION HUMANIZED MONOCLONAL ANTI-VEGF ANTIBODY ANGIOGENESIS INHIBITOR", DRUGS OF THE FUTURE, PROUS SCIENCE, ES, vol. 28, no. 6, 1 June 2003 (2003-06-01), pages 541-545, XP008077008, ISSN: 0377-8282, DOI: 10.1358/DOF.2003.028.06.738510
- MCCONNELL VIVIENNE ET AL: "Assessment of a putative locus for exdative age-related macular degeneration on chromosome 16p", JOURNAL OF MEDICAL GENETICS,JOURNAL OF MEDICAL GENETICS CONF- BRITISH HUMAN GENETICS CONFERENCE; WARWICK, UK; SEPTEMBER 06 -08, 2010; BRITISH HUMAN GENETICS CONFERENCE, BMJ PUBLISHING GROUP, LONDON, GB; YORK, ENGLAND, [Online] vol. 41, no. Suppl. 1, 3.10, 15 September 2004 (2004-09-15), page S65, XP008162331, ISSN: 0022-2593 Retrieved from the Internet: URL:http://main03.internal.epo.org/project s/pddoc/evl.nsf/J_BMJ?OpenPage>
- MORALES SHAWN A ET AL: "Epithelial Membrane Protein 2 Controls VEGF Expression in ARPE-19 Cells", IOVS, vol. 54, no. 3, March 2013 (2013-03), pages 2367-2372, XP008162318,

## Description

### CROSS-REFERENCES TO RELATED APPLICATIONS

### BACKGROUND

In the industrialized world the average life expectancy is over 80 years of age and is increasing steadily. Unfortunately, the quality of life for the elderly is often dramatically decreased by the ocular condition known as age related macular degeneration ("ARMD" or "AMD"). The risk of the disease reaches more than 5% for individuals older than 80.

AMD is the leading cause of blindness in Americans over the age of 60 and the third leading cause of worldwide blindness. The World Health Organization has estimated that about 14 million people are blind or severely impaired because of AMD. The affliction of AMD has great impact on the physical and mental health of the geriatric population and their families and presents a significant public health care burden.

There are two forms of AMD, atrophic or dry AMD and neovascular or wet (exudative) AMD. Typically AMD begins as dry AMD. Dry AMD is characterized by the formation of yellow plaque like deposits called drusen in the macula, between the retinal pigment epithelium (RPE) and the underlying choroid. About 15% of dry AMD patients develop wet AMD, which leads to severe vision loss.

The severe vision loss associated with wet AMD is caused by the growth of abnormal new blood vessels from the choriocapillaris, a process call choroidal neovascularization (CNV). The new vessels tend to bleed, exude serum and promote excessive reparative responses within the macula. These changes, in turn, alter the anatomical relationship between the overlying neurosensory retina and the underlying RPE layer, causing detachment, dysfunction and degeneration of the photoreceptors. In the most severe cases, participants lose the ability to read or perform activities of daily living without aid.

AMD prophylaxis and therapy has changed radically over the past decade, beginning with the Age-Related Eye Disease Study, which in 2001 described a reduction in the risk for AMD progression of 25% in individuals who used vitamin supplements containing antioxidants and zinc.

Currently there are only four treatments approved by the FDA for wet AMD: laser surgery, photodynamic therapy (PDT), and the drugs Macugen®, pegaptanib sodium and Lucentis™ (ranibizumab) intravitreal injections. Laser, PDT and pegaptanib may slow the rate of vision decline and/or stop vision loss.

Laser surgery attempts to destroy the fragile, leaky blood vessels using a high energy beam of light. This treatment, however, may also destroy some surrounding healthy tissue and therefore actually contribute to further vision loss. Because of this, only a small percentage of people with wet AMD can be treated with laser surgery.

Photodynamic therapy also attempts to destroy the newly formed blood vessels in the patient's eye. Verteporfin (Visudyne®) is injected into the patient's arm. The drug travels through the patient's body, "sticking" to the surface of new blood vessels. A light is then shone in the patient's eye, which activates the drug, which in turn destroys the new blood vessel. Photodynamic therapy merely temporarily slows the rate of vision loss; it does not stop vision loss or restore vision. Moreover, because the drug is activated by light, the patient must avoid sunlight and bright indoor lights for five days after treatment.

The neovasculariztion associated with AMD was first effectively treated with photodynamic therapy, but actual improvements in vision were not achieved until the use of intraocular injections of anti-VEGF (vascular endothelial growth factor) reagents. Pegaptanib (Macugen®, Eyetech Pharmaceuticals Inc. and Pfizer Inc.), is approved for treatment of wet AMD is a pegylated oligonucleotide aptamer targeting VEGF. Ranibizumab (Lucentis™), an antibody fragment targeting VEGF, has recently been approved by FDA for the treatment of wet AMD.

Unfortunately, anti-VEGF therapy suffers from a significant drawback; the retina requires some levels of VEGF to be present for normal function. Studies have demonstated adverse affect to the retina due to low VEGF levels following anti-VEGF treatment. Thus, a need remains for methods and compositions which are useful in the treatment and/or prevention of AMD and other afflictions of the eye.

The 4-transmembrane (tetraspan) protein EMP2 (Epithelial Membrane Protein 2) is expressed at discrete locations in the eye. In the eye, EMP2 is localized to multiple epithelial layers including the cornea, ciliary boding, and RPE. The use of an inhibitory anti-EMP2 antibody or an EMP2 si-RNA in the treatment of the eye disease conjunctivitis is described in WO2009/048980 and in the treatment of proliferative vitreoretinopathy (PVR) is described on WO2011 /063161. Morales et al. (Investigate ophthalmology and visual science, association for research in vision and ophthalmology, US, vol 50. No. 1, pages 462-469, 1 January 2009) discloses that EMP2 si-RNA descreases the level of EMP2 expressed in the retinal pigment epithelium cell line ARPE-19 and that an EMP2 ribozyme decreases the level of EMP2 and collagen gel contraction of ARPE-19 cells which is an in vitro correlate of the eye disease proliferative vitreoretinopathy (PVR). The medical use of the anti-VEGF antibody, ranibizumab, an angiogenesis inhibitor, in the treatment of wet age related macular degeneration is described in Sorbera et al. (Drugs of the future, Prous Science, ES, vol. 28, no. 6, pages 541-545, 1 June 2003).

This invention provides novel methods for treating and/or preventing AMD and other affictions of the eye using reagents that bind to or regulate EMP2 protein or EMP2 nucleic acid.

### BRIEF SUMMARY OF THE INVENTION

The instant invention provides an epithelial membrane protein 2 (EMP2) inhibitor for use in treating wet age related macular degeneration (AMD), wherein the EMP2 inhibitor is selected from a group consisting of an anti-EMP2 antibody, an EMP2 si-RNA, and combinations thereofIn some embodiments, the EMP2 inhibitor is an antibody. These antibodies are capable of specifically binding to EMP2. In addition, the disclosed anti-EMP2 antibodies can be polyclonal or monoclonal. Further, the anti-EMP2 antibodies may be a human antibody, a humanized antibody a chimeric antibody, a recominant antibody, a diabody, minibody, triabody, or an antibody fragment having the light and heavy chain variable sequences or CDRs corresponding to KS49, KS83, KS41, and KS89.

In some embodiments, the anti-EMP2 antibody encompasses a heavy chain and a light chain, wherein the heavy chain encompasses three complementarity determining regions (CDRs): HCDR1, HCDR2, and HCDR3, and wherein the light chain comprises three CDRs: LCDR1, LCDR2, and LCDR3, wherein HCDR1 encompasses SEQ ID NO:8, HCDR2 encompasses SEQ ID NO:9, and HCDR3 encompasses SEQ ID NO:10, and wherein LCDR1 encompasses SEQ ID NO:12, LCDR2 encompasses SEQ ID NO:13, and LCDR3 encompasses SEQ ID NO:14.

In other embodiments, the invention provides for treating AMD wherein the EMP2 inhibitor is an EMP2 si-RNA. In some embodiments, the EMP2 si-RNA encompasses a target sequence wherein the target sequence encompasses SEQ ID NO:3, SEQ ID NO:4, SEQ ID NO:5, or SEQ ID NO:6.

In other embodiments, the EMP2 si-RNA encompasses a target sequence selected from the group: SEQ ID NO:3, SEQ ID NO:4, SEQ ID NO:5, and SEQ ID NO:6.

The EMP2 inhibitors may be formulated into pharmaceutical compositions. These pharmaceutical compositions may be suitable for adminstration to the eye. The EMP2 inhibitor may be administered to the eye by an intraocular route. The EMP2 inhibitor may be administered topically or subconjunctivally. These compositions are provided in an effective amount.

The invention further provides an epithelial membrane protein 2 (EMP2) inhibitor for use in treating a disease of the eye, wherein the disease of the eye includes diabetic retinopathy, corneal neovascularization, choroidal neovascularization, cyclitis, Hippel-Lindau Disease, retinopathy of prematurity, pterygium, histoplasmosis, iris neovascularization, macular edema, glaucoma-associated neovascularization, and Purtscher's retinopathy and the EMP2 inhibitor is selected from a group consisting of an anti-EMP2 antibody, an EMP2 si-RNA, and combinations thereof..

The invention further provides an epithelial membrane protein 2 (EMP2) inhibitor for use in reducing the expression of vascular endothelial growth factor (VEGF) in the eye of a patient suffering from a disease selected from the group consisting of wet age related macular degeneration (AMD), diabetic retinopathy, corneal neovascularization, choroidal neovascularization, cyclitis, Hippel-Lindau Disease, retinopathy of prematurity, pterygium, histoplasmosis, iris neovascularization, macular edema, glaucoma-associated neovascularization, and Purtscher's retinopathy, wherein the EMP2 inhibitor is selected from a group consisting of an anti-EMP2 antibody, an EMP2 si-RNA, and combinations thereof. In some embodiments, the use for reducing the expression of VEGF in the eye entails the use of an anti-EMP2 antibody. In other embodiments, the EMP2 inhibitor is an EMP2 si-RNA.

In some embodiments, the EMP2 inhibitor for use in treating wet AMD comprises at least two epithelial membrane protein 2 (EMP2) inhibitors. In some embodiments, the two EMP2 inhibitors are an anti-EMP2 antibody and an EMP2 si-RNA.

### BRIEF DESCRIPTION OF THE DRAWINGS

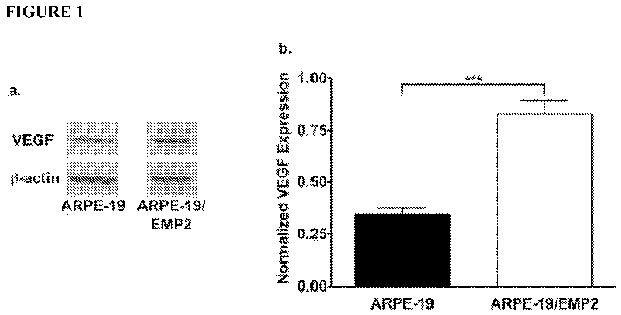

FIG. 1 demonstrates that EMP2 over-expression increases VEGF expression. Cell extracts fractionated by 4-20% SDS-PAGE gradient gel in reducing conditions. (A) Representative immunoblots. (B) Band density, normalized to the β-actin loading control, was quantified.

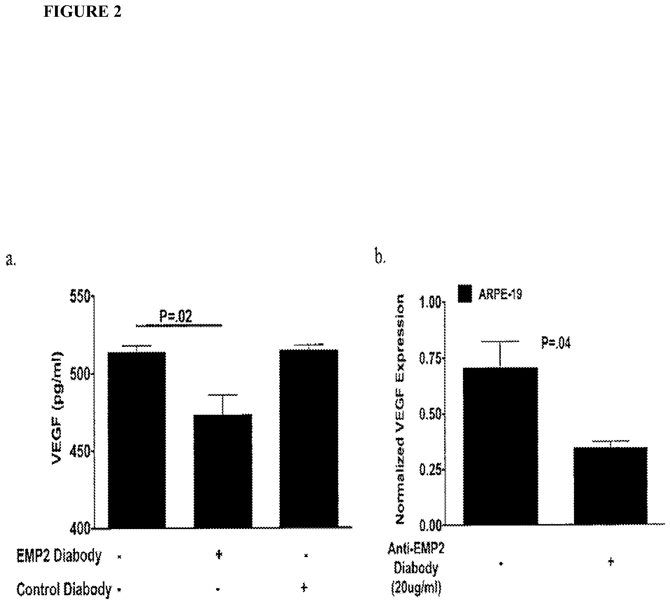

FIG. 2 demonstrates the effect of anti-EMP2 diabodies on VEGF expression. (A) Anti-EMP2 treatment significantly decreases VEGF levels (middle bar) as reveled by ELISA as compared to untreated (left bar) and control diabody (right bar). (B) Results of a Western blot analysis of VEGF expression following treatment with anti-EMP2 diabodies. Anti-EMP2 diabody treatment (right bar) significantly decreases VEGF levels as relative to untreated cells.

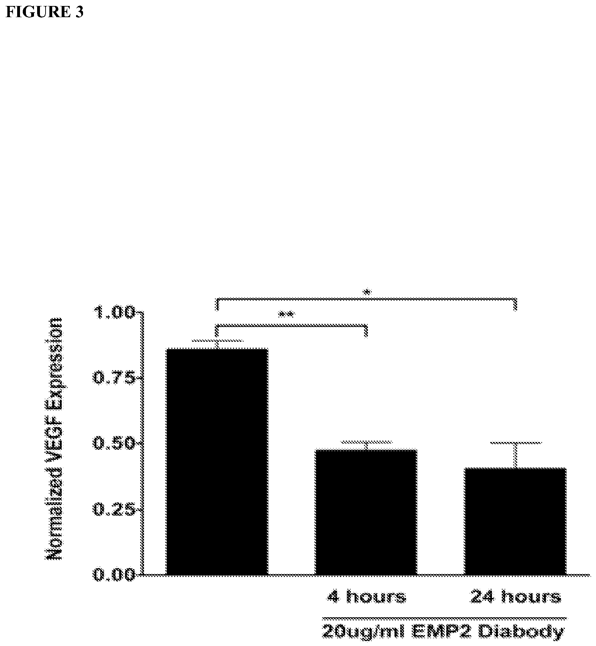

FIG. 3 demonstrates a time course of VEGF reduction following anti-EMP2 diabody treatment.

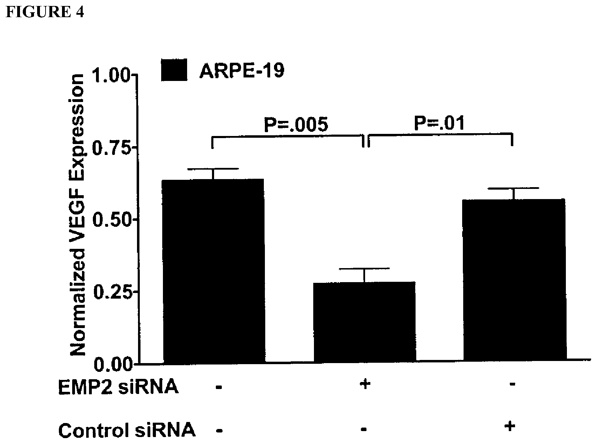

FIG. 4 demonstrates that si-RNA for EMP2 decreases VEGF expression. Cells treated with EMP2 specific si-RNA (middle bar) showed a significant decrease in VEGF expressio as compared to untreated cells (left bar) or cells treated with control si-RNA (right bar).

### DETAILED DESCRIPTION OF THE INVENTION

### INTRODUCTION

Age-related macular degenertion ("ARMD" or "AMD") is a complex disease whose risk factors include aging, family history of AMD, smoking, hypertension, obesity, diet, and ethnicity, and there is a strong indication of a genetic contribution. Ambati et al., Surv. Ophalmol., 48:257 (2003). In the United States more than 50% of the cases of visual impairment occur secondary to a disease refered to as AMD. Two major clinical phenotypes of AMD are recognized: a nonexudative (thy) type and an exudative (wet) type. The most severe form of AMD, wet AMD results from pathological neovascularization of the retina called choroidal or subretinal neovasculariztion.

AMD is associated with changes to the retinal pigment epithelium (RPE). RPE is a monolayer of cuboidal, polarized epithelium with close contact to the neurosensory retina at the apical membrane. RPE is considered pivotal in maintaining retinal homeostasis and normal retinal functions. RPE is also responsible for the regulation of inflammatory response in the ocular microenvironment and for the maintaince of the blood brain barrier. It is believed that RPE is responsible for production of VEGF, a step in AMD pathogenesis.

EMP2 (epithelial membrane protein 2), which is relatively highly expressed in RPE, is a member of the growth arrest specific gene 3/peripheral mylein protein 22 (GAS3/PMP22) group of the tetraspan protein superfamily. EMP2 has at least two supposed biochemical roles in receptor-mediated cellular behavior. First, EMP2 regulates trafficking and intracellular compartmetalization and surface display of selected receptors and glycolipids. Second, EMP2 physically associates with and is thought to regulate the activity of integrin-FAK (focal adhesion kinase) signaling complexes.

The instant disclosure provides methods and compositions for treating ocular conditions and diseases using an effective amount of EMP2 inhibitors.

An "EMP2 inhibitor" refers to an agent that inhibits a function, activity, or levels of EMP2 using assays known in the art. An EMP2 inhibitor can be an EMP2 polypeptide; an anti-EMP2 antibody; an EMP2 siRNA molecule; an EMP2 aptamer; an EMP2-ribozyme; a compound which competes with binding of to EMP2, or an agent or compound which inhibits the expression, transcription, or translation of EMP2 nucleic acids in a host cell.

An EMP2 inhibitor can also reduce the function, activity or expression levels of VEGF. For example, an EMP2 inhibitor can reduce the presence of VEGF protein in a system by 2%, 5%, 10%, 15%, 20%, 25%, 30%, 35%, 40%, 45%, 50%, 55%, 60%, 65%, 70%, 75%, 80%, 85%, 90%, 95%, 98%, 99%, or more.

An "EMP2 polypeptide" refers to a conservatively modified variant of a polypeptide of SEQ ID NO: 1. Accordingly, the EMP polypeptide may consist of the sequence of EMP2 of SEQ ID NO:1 or a fragment thereof. The fragment may be from 15 to 25, 15 to 40, 25 to 50, 50 to 100 amino acids long, or longer. The fragment may correspond to that of EMP2 from position 16 to 64 of SEQ ID NO: 1.

A "polypeptide," "peptide, and "protein" are used interchangeably and refer to a polymer of amino acid residues. Methods for obtaining (e.g., producing, isolating, purifying, synthesizing, and recombinantly manufacturing) polypeptides are well known to one of ordinary skill in the art.

"Amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ- carboxyglutamate, and O-phosphoserine.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

The following eight groups each contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (see, e.g. , Creighton, Proteins (1984)).

### EMP2 Inhibitors

Asays for identifying EMP2 inhibitors of the invention are conducted in the presence of the candidate inhibitor and then the results are compared to control samples without the inhibitor to examine for the desired activity or to determine the functional effect of the candidate inhibitor. A positive reference control, which is an agent having the desired activity, may be used.

The inhibition may take place *in vivo* or *in vitro,* and is accomplished by contacting a cell with a composition comprising an EMP2 inhibitor. Contacting *in vitro* may be accomplished, for example, by adding the EMP2 inhibitor to a cell culture medium. Contacting *in vivo* may be accomplished by administering a sterile or pharmaceutically acceptable composition with the EMP2 inhibitor to an animal (e.g., patient).

"Determining the functional effect" refers to assaying for a compound that increases or decreases a parameter that is indirectly or directly under the influence of a polynucleotide or polypeptide for use according to the invention, e.g., measuring physical and chemical or phenotypic effects.

Such functional effects can be measured by any means known to those skilled in the art, e.g., changes in spectroscopic (e.g., fluorescence, absorbance, refractive index), hydrodynamic (e.g., shape), chromatographic, or solubility properties for the protein; measuring inducible markers or transcriptional activation of the protein; measuring binding activity or binding assays, e.g. binding to antibodies; measuring changes in ligand binding affinity; e.g., via chemiluminescence, fluorescence, colorimetric reactions, antibody binding, inducible markers, and ligand binding assays.

A measure of EMP2 inhibition is a reduction in VEGF expression in cells contacted with an EMP2 inhibitor. Accordingly, inhibitors of EMP2 can be identified by contacting cells overexpressing EMP2 with an inhibitor and measuring a functional effect, for example VEGF expression levels. In some instances, such assays measure the presence of VEGF protein, for example by ELISA or Western blot. In other instances, such assays measure the presence of VEGF mRNA. Other suitable methods for measuring VEGF polypeptide and/or mRNA are known to those of skill in the art.

In some instances, when determining a functional effect, control samples can be assigned a relative of 100%. Inhibition is achieved when the assayed function, activity or expression level is reduced by 2%, 5%, 10%, 15%, 20%, 25%, 30%, 35%, 40%, 45%, 50%, 55%, 60%, 65%, 70%, 75%, 80%, 85%, 90%, 95%, 98%, 99%, or more relative to the control.

Suitable methods for identifying inhibitors are set forth in the Examples.

### Antibodies

In some embodiments, the EMP2 inhibitor is an antibody. An "anti-EMP2 antibody" or "EMP2 antibody" according to the invention is an antibody which can specifically bind to the EMP2 polypeptide of SEQ ID NO:1.

The antibodies include recombinant antibodies, polyclonal antibodies, monoclonal antibodies, chimeric antibodies, human monoclonal antibodies, humanized or primatized monoclonal antibodies, and antibody fragments. The antibodies may bind to an external loop sequence of EMP2.

"Antibody" refers to a polypeptide comprising a framework region from an immunoglobulin gene or fragments thereof that specifically binds and recognizes an antigen. The recognized immunoglobulin genes include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Typically, the antigen-binding region of an antibody provides specificity and affinity of binding.

An exemplary immunoglobulin (antibody) structural unit comprises a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (V_{L}) and variable heavy chain (V_{H}) refer to these light and heavy chains respectively.

Antibodies exist, e.g., as intact immunoglobulins or as a number of well-characterized fragments produced by digestion with various peptidases. Thus, for example, pepsin digests an antibody below the disulfide linkages in the hinge region to produce F(ab)'₂ a dimer of Fab which itself is a light chain joined to V_{H}-C_{H}I by a disulfide bond. The F(ab)'₂ may be reduced under mild conditions to break the disulfide linkage in the hinge region, thereby converting the F(ab)'₂ dimer into an Fab' monomer. The Fab' monomer is essentially Fab with part of the hinge region (see Fundamental Immunology (Paul ed., 3d ed. 1993).

While various antibody fragments are defined in terms of the digestion of an intact antibody, one of skill will appreciate that such fragments may be synthesized *de novo* either chemically or by using recombinant DNA methodology. Thus, the term antibody, also includes antibody fragments either produced by the modification of whole antibodies, or those synthesized de novo using recombinant DNA methodologies (e.g., single chain Fv) or those identified using phage display libraries (see, for example, McCafferty et al., Nature 348:552-554 (1990)).

Accordingly, the term antibody also embraces minibodies, diabodies, triabodies and the like. "Diabodies" referes to small bivalent biospecific antibody fragments with high avidity and specificity. Their high signal to noise ratio is typically better due to a better specificity and fast blood clearance increasing their potential for diagnostic and therapeutic targeting of specific antigen (Sundaresan et al., J Nucl Med 44:1962-9 (2003).

In addition, these antibodies are advantageous because they can be engineered if necessary as different types of antibody fragments ranging from a small single chain Fv to an intact IgG with varying isoforms (Wu & Senter, Nat.Biotechnol. 23:1 137-1146 (2005)). In some embodiments, the antibody fragment is part of a diabody. The following human-origin antibody sequences encode for high-avidity antibodies specific for human (KS49, KS83) and mouse (KS83) EMP2 and have antibody variable region heavy and light chains suitable for use in either aspect of the invention.

Anti-EMP-2 variable region sequences, used to encode proteins on backbones including for native antibody, fragment antibody, or synthetic backbones, can avidly bind EMP-2. Via this binding, these proteins can be used for EMP-2 detection, and to block EMP- 2 function.

Antibodies (e.g., diabodies, minibodies, triabodies) or fragments thereof may have the CDRs of a diabody selected from KS49, KS83, KS41, and KS89. These antibodies may lack the polyhistine tag. The diabodies may possess the light and heavy chain of a KS49, KS83, KS41, or KS89 diabody. The antibodies may be substantially identical in sequence to a diabody selected from the group consisting of KS49, KS83, KS41, and KS89 with or without the polyhistidine tag. The antibodies may be substantially identical in sequence to the light and heavy chain sequences of a diabody selected from the group consisting of KS49, KS83, KS41, and KS89. These identities can be 65%, 70%, 75%, 80%, 85%, 90%, and preferably 91%, 92%, 93%, 94%, 95%, 96%, 97%, 98% or 99% or greater amino acid sequence identity. The antibodies may comprise CDRs sequences identical to those of the KS49, KS83, KS41, or KS89 diabody.

The present invention may provide anti-EMP-2 sequences comprising CDR regions of an antibody selected from KS49, KS83, KS41, and KS89. The CDR regions may be used to construct an anti-EMP-2 binding protein, including without limitation, an antibody, a scFv, a triabody, a diabody, a minibody, and the like.

An anti-EMP-2 binding protein may comprise at least one CDR region from an antibody selected from KS49, KS83, KS41, and KS89. Anti-EMP-2 binding proteins may comprise, for example, a CDR- H1, a CDR-H2, a CDR-H3, a CDR-L1, a CDR-L2, a CDR-L3, or combinations thereof, from an antibody provided herein. An anti-EMP-2 binding protein may comprise all three CDR-H sequences of an antibody provided herein, all three CDR-L sequences of an antibody provided herein, or both.

The phrase "specifically (or selectively) binds" to an antibody or "specifically (or selectively) immunoreactive with," when referring to a protein or peptide, refers to a binding reaction that is determinative of the presence of the protein, often in a heterogeneous population of proteins and other biologies.

Thus, under designated immunoassay conditions, the specified antibodies bind to a particular protein at least two times the background and more typically more than 10 to 100 times background. Specific binding to an antibody under such conditions requires an antibody that is selected for its specificity for a particular protein.

For example, polyclonal antibodies can be selected to obtain only those polyclonal antibodies that are specifically immunoreactive with the selected antigen and not with other proteins. This selection may be achieved by subtracting out antibodies that cross-react with other molecules. A variety of immunoassay formats may be used to select antibodies specifically immunoreactive with a particular protein. For example, solid-phase ELISA immunoassays are routinely used to select antibodies specifically immunoreactive with a protein (see, e.g., Harlow & Lane, Using Antibodies, A Laboratory Manual (1998) for a description of immunoassay formats and conditions that can be used to determine specific immunoreactivity) .

For example, rabbit polyclonal antibodies to EMP2 are known in the art (see, Wang et al., Blood 97:3890-3895 (2001)). Such antibodies may be obtained using glutathione-S- transferase-EMP2 fusion proteins. Rabbit antibodies can be generated against the first extracellular region of the gene (from amino acid 16 to 64) constructed as a glutathione-5- transferase (GST)-EMP2 fusion protein. The EMP2 peptide can be cloned by PCR using the following primers: CGCGGATCCTCTACCATTGACAATGCCTGG (forward; BamH1 underlined); CCGGAATTCTTACGCCTGCATCACAGAATAACC (reverse, EcoR1 underlined).

The PCR product can be directionally cloned into the BamHI and EcoRI sites of the pGEX-4T-1 vector that contains GST gene (Pharmacia). The EMP2 fragment is cloned in frame with the GST to create a fusion protein. The insert can be confirmed by sequencing. The GST fusion protein can be produced as previously described (see, Smith DB et al., Gene 67:31-40 (1988)). Bacteria in log phase (OD₆₀₀ 0.6 to 0.9) can be induced for 2.5 to 3 hours at 37°C with 1 mM isopropyl-1-thio-3-D-galactopyranoside. Bacteria are lysed, and the soluble fraction loaded onto a glutathione-Sepharose column (Pierce, Rockford, IL). The columns are washed with 10 bed volumes of phosphate-buffered saline (PBS)/EDTA. The fusion protein elutes from the column using 20 mM reduced glutathione (Sigma, St Louis, MO) in 50 mM Tris-Cl, pH 8.0. For antibody preparation, rabbits are immunized twice with the GST-EMP2 fusion protein, and serum is collected, starting 2 weeks after the last immunization (Research Genetics, Huntsville, AL).

Anti-EMP2 CDR sequences may be used on an antibody backbone, or fragment thereof, and likewise may include humanized antibodies, or antibodies containing humanized sequences. These antibodies may be used, for example, to detect EMP-2, to detect cells expressing EMP-2 in vivo, or to block EMP-2 function. The CDR regions may be defined using the Kabat definition, the Chothia definition, the AbM definition, the contact definition, or any other suitable CDR numbering system.

Suitable methods for identifying antibody inhibitors for use according to the invention are set forth in Examples 4 and 6.

### EMP2 si-RNA

In some embodiments, the EMP2 inhibitor is an EMP2 siRNA. An "siRNA" or "RNAi" refers to a nucleic acid that forms a double stranded RNA, which double stranded RNA has the ability to reduce or inhibit expression of a gene or target gene when the siRNA expressed in the same cell as the gene or target gene. "siRNA" or "RNAi" thus refers to the double stranded RNA. formed by the complementary strands.

A method for evualating siRNA molecules for EMP2 inhibition can involve, for example, the addition of a candidate siRNA specific for EMP2 to test sample. Control samples can include, for example, samples in which no siRNA is added and/or samples in which irrelevant (not specific for EMP2) siRNA is added. Inhibition of EMP2 or VEGF in the test sample relative to control sample(s) is indicative of a siRNA EMP2 inhibitor.

Suitable methods for identifying EMP2 siRNA inhibtors are provided in Examples 4, 5, and 6.

The complementary portions of the siRNA that hybridize to form the double stranded molecule typically have substantial or complete identity. An siRNA may refer to a nucleic acid that has substantial or complete identity to a target gene and forms a double stranded siRNA. Typically, the siRNA is at least about 15-50 nucleotides in length (e.g., each complementary sequence of the double stranded siRNA is 15-50 nucleotides in length, and the double stranded siRNA is about 15-50 base pairs in length, preferable about preferably about 20-30 base nucleotides, preferably about 20-25 or about 24-29 nucleotides in length, e.g., 20, 21, 22, 23, 24, 25, 26, 27, 28, 29, or 30 nucleotides in length.

The design and making of siRNA molecules and vectors are well known to those of ordinary skill in the art. For instance, an efficient process for designing a suitable siRNA is to start at the AUG start codon of the mRNA transcript and scan for AA dinucleotide sequences (see, Elbashir et al., EMBO J 20:6877-6888 (2001)). Each AA and the 3' adjacent nucleotides are potential siRNA target sites. The length of the adjacent site sequence will determine the length of the siRNA. For instance, 19 adjacent sites would give a 21 Nucleotide long siRNA. This approach is also compatible with using RNA pol III to transcribe hairpin siRNAs. RNA pol III terminates transcription at 4-6 nucleotide poly(T) tracts to create RNA molecules having a short poly(U) tail. However, siRNAs with other 3' terminal dinucleotide overhangs can also effectively induce RNAi and the sequence may be empirically selected. For selectivity, target sequences with more than 16-17 contiguous base pairs of homology to other coding sequences can be avoided by conducting a BLAST search (see, world wide web.ncbi.nlm.nih.gov/BLAST).

The siRNA can be administered directly or siRNA expression vectors can be used to induce RNAi. A vector can have inserted two inverted repeats separated by a short spacer sequence and ending with a string of T's which serve to terminate transcription. The expressed RNA transcript is predicted to fold into a short hairpin siRNA . The selection of siRNA target sequence, the length of the inverted repeats that encode the stem of a putative hairpin, the order of the inverted repeats, the length and composition of the spacer sequence that encodes the loop of the hairpin, and the presence or absence of 5'-overhangs, can vary. A preferred order of the siRNA expression cassette is sense strand, short spacer, and antisense strand. Hairpin siRNAs with these various stem lengths (e.g., 15 to 30) are suitable. The length of the loops linking sense and antisense strands of the hairpin siRNA can have varying lengths (e.g., 3 to 9 nucleotides, or longer). The vectors may contain promoters and expression enhancers or other regulatory elements which are operably linked to the nucleotide sequence encoding the siRNA.

The expression "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers. These control elements may be designed to allow the clinician to turn off or on the expression of the gene by adding or controlling external factors to which the regulatory elements are responsive.

The EMP2 inhibitor maybe an EMP2 ribozyme which can inhibit the expression of EMP2 when present in a cell. Ribozymes are enzymatic RNA molecules capable of catalysing the specific cleavage of RNA. The mechanism of ribozyme action involves sequence specific hybridisation of the ribozyme molecule to complementary target RNA, followed by an endonucleolytic cleavage. Within the scope of the invention are engineered hammerhead motif ribozyme molecules that specifically and efficiently catalyse endonucleolytic cleavage of EMP2 mRNA. Specific ribozyme cleavage sites within any potential RNA target are initially identified by scanning the target molecule for ribozyme cleavage sites, which include the following sequences, GUA, GUU and GUC. Once identified, short RNA sequences of between 15 and 20 ribonucleotides corresponding to the region of the target gene containing the cleavage site may be evaluated for predicted structural features such as secondary structure that may render the oligonucleotide sequence unsuitable.

DNA molecules and ribozymes of the invention may be prepared by any method known in the art for the synthesis of RNA molecules. These include techniques for chemically synthesizing oligodeoxyribonucleotides well known in the art such as for example solid phase phosphoramidite chemical synthesis.

Alternatively, RNA molecules may be generated by *in vitro* and *in vivo* transcription of DNA sequences encoding the antisense RNA molecule. Such DNA sequences may be incorporated into a wide variety of vectors, which incorporate suitable RNA polymerase promoters such as the T7 or SP6 polymerase promoters. Methods of making ribozymes are well known in the art (see, for instance, U.S. Patent Application Publication No. 20060062785).

Construction of suitable vectors for the EMP2 siRNA or EMP2 Ribozymes containing the desired EMP2 siRNA or EMP2 Ribozyme sequences and control sequences employs standard ligation and restriction techniques, which are well understood in the art (see Maniatis et al., in Molecular Cloning: A Laboratory Manual, Cold Spring Harbor Laboratory, New York (1982)). Isolated plasmids, DNA sequences, or synthesized oligonucleotides are cleaved, tailored, and religated in the form desired.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine.

Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid which encodes a polypeptide is implicit in each described sequence with respect to the expression product, but not with respect to actual probe sequences.

The terms "identical" or percent "identity," in the context of two or more nucleic acids or polypeptide sequences, including EMP2 siRNA and EMP2 polypeptides, refer to two or more sequences or subsequences that are the same or have a specified percentage of amino acid residues or nucleotides that are the same (i.e., about 60% identity, preferably 65%, 70%, 75%, 80%, 85%, 90%, 91 %, 92%, 93%, 94%, 95%, 96%, 97%, 98%, 99%, or higher identity over a specified region, when compared and aligned for maximum correspondence over a comparison window or designated region) as measured using a BLAST or BLAST 2.0 sequence comparison algorithms with default parameters described below, or by manual alignment and visual inspection (see, e.g., NCBI web site hypertext transfer protocol://www.ncbi.nlm.nih.gov/BLAST/ or the like).

Such sequences are then said to be "substantially identical." This also refers to, or may be applied to, the compliment of a test sequence. This also includes sequences that have deletions and/or additions, as well as those that have substitutions. Preferably, identity exists over a region that is at least about 25 amino acids or nucleotides in length, or more preferably over a region that is 50-100 amino acids or nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

A "comparison window," as used herein, includes reference to a segment of any one of the number of contiguous positions selected from the group consisting of from 20 to the full length of the reference sequence, usually about 25 to 100, or 50 to about 150, more usually about 100 to about 150 in which a sequence may be compared to a reference sequence of the same number of contiguous positions after the two sequences are optimally aligned. Methods of alignment of sequences for comparison are well-known in the art.

Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat 7. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection {see, e.g., Current Protocols in Molecular Biology (Ausubel et al., eds. 1995 supplement)).

A preferred example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. MoI. Biol. 215:403-410 (1990), respectively. BLAST and BLAST 2.0 are used, with the parameters described herein, to determine percent sequence identity for the nucleic acids and proteins of the invention. Software for performing BLAST analyses is publicly available through the National Center for Biotechnology Information (hypertext transfer protocol://www.ncbi.nlm.nih.gov/). This algorithm involves first identifying high scoring sequence pairs (HSPs) by identifying short words of length W in the query sequence, which either match or satisfy some positive- valued threshold score T when aligned with a word of the same length in a database sequence. T is referred to as the neighborhood word score threshold (Altschul et al., supra). These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, M=5, N=-4 and a comparison of both strands. For amino acid sequences, the BLASTP program uses as defaults a wordlength of 3, and expectation (E) of 10, and the BLOSUM62 scoring matrix (see Henikoff & Henikoff, Proc. Natl. Acad. Sci. USA 89:10915 (1989)) alignments (B) of 50, expectation (E) of 10, M=5, N=-4, and a comparison of both strands.

"Nucleic acid" refers to deoxyribonucleotides or ribonucleotides and polymers thereof in either single- or double-stranded form, and complements thereof. The term encompasses nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphorothioates, phosphoramidates, methyl phosphonates, chiral-methyl phosphonates, 2-O-methyl ribonucleotides, peptide-nucleic acids (PNAs).

Unless otherwise indicated, a particular nucleic acid sequence also encompasses conservatively modified variants thereof (e.g., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); Rossolini et al., MoI. Cell. Probes 8:91-98 (1994)). The term nucleic acid is used interchangeably with gene, cDNA, mRNA, oligonucleotide, and polynucleotide.

"Heterologous" refers to portions of a nucleic acid indicates that the nucleic acid comprises two or more subsequences that are not found in the same relationship to each other in nature. For instance, the nucleic acid is typically recombinantly produced, having two or more sequences from unrelated genes arranged to make a new functional nucleic acid, e.g., a promoter from one source and a coding region from another source. Similarly, a heterologous protein indicates that the protein comprises two or more subsequences that are not found in the same relationship to each other in nature (e.g., a fusion protein).

### Ocular Conditions and Diseases

An "ocular condition" can include a disease or aliment that affects or involves the eye or one of the parts or regions of the eye. Broadly speaking the eye includes the eyeball and the tissues and fluids which constitute the eyeball, the periocular muscles (such as the oblique and rectus muscles) and the portion of the optic nerve which is within or adjacent to the eyeball.

A "front of the eye" or "anterior ocular condition" is a disease or ailment that affects or which involves an ocular region or site, such as a periocular muscle, an eye lid or an eye ball tissue or fluid which is located anterior to the posterior wall of the lens capsule or ciliary muscles. Thus, a front of the eye ocular condition affects or involves, the conjunctiva, the cornea, the conjunctiva, the anterior chamber, the iris, the lens and the lens capsule as well as blood vessels, lymphatics and nerves which vascularize, maintain or innervate an anterior ocular region or site, or the like.

A front of the eye (anterior) ocular condition can include a disease, ailment or condition, such as for example, aphakia; pseudophakia; astigmatism; blepharospasm; cataract; conjunctival diseases; conjunctivitis; corneal diseases; corneal ulcer; dry eye syndromes; eyelid diseases; lacrimal apparatus diseases; lacrimal duct obstruction; myopia; presbyopia; pupil disorders; refractive disorders and strabismus. Glaucoma can be considered to be a front of the eye ocular condition because a clinical goal of glaucoma treatment can be to reduce a hypertension of aqueous fluid in the anterior chamber of the eye (i.e. reduce intraocular pressure).

A "posterior ocular condition" (or back of the eye) is a disease or ailment that primarily affects or involves a posterior ocular region or site such as, for example, choroid or sclera (in a position posterior to a plane through the posterior wall of the lens capsule), vitreous, vitreous chamber, retina, optic nerve (i.e. the optic disc), and blood vessels and nerves which vascularize or innervate a posterior ocular region or site or the like.

Thus, a posterior ocular condition can include a disease or ailment, such as, for example, macular degeneration (such as non-exudative age related macular degeneration and exudative age related macular degeneration); choroidal or retinal neovascularization. Glaucoma can also be considered a back of the eye ocular condition because of the damage to the optic nerve.

An "intraocular neovascular disease" is a disease characterized by ocular neovascularization. Examples of intraocular neovascular diseases include, but are not limited to, e.g., proliferative retinopathies, choroidal neovascularization (CNV), age-related macular degeneration (AMD), diabetic and other ischemia-related retinopathies, diabetic macular edema, pathological myopia, von Hippel-Lindau disease, histoplasmosis of the eye, corneal neovascularization, retinal neovascularization.

"Dry AMD" (also referred to as atrophic age related macular degeneration) refers to a retinal condition in which drusen are present in the macula but with little or no retinal neovascularization.

The dry form of AMD is associated with cell death of the light-sensitive macular part of the retina, which is required for fine vision used in activities such as reading, driving or recognizing faces. Over time, as less of the macula functions, central vision in the affected eye can be lost gradually. One of the most common early signs of dry AMD is the appearance of drusen.

Drusen are yellow deposits under the retina and are often found in people over the age of 60.

Early and intermediate AMD are characterized by the presence of small or medium-sized drusen, and persons suffering from early and intermediate AMD may require additional light when reading and experience a blurred spot in the center of their vision. Persons suffering from advanced AMD, in addition to the presence of medium or large-sized drusen, exhibit a breakdown of light-sensitive cells and supporting tissue in the central retinal area.

"Wet AMD" refers to a retinal condition characterized by the presence of retinal neovascularization (category 4 or advanced AMD) or vision loss.

Posterior ocular conditions also can include acute macular neuroretinopathy; macular edema (such as cystoid macular edema and diabetic macular edema); Behcet's disease, retinal disorders, diabetic retinopathy (including proliferative diabetic retinopathy); retinal arterial occlusive disease; central retinal vein occlusion; uveitic retinal disease; retinal detachment; ocular trauma which affects a posterior ocular site or location; a posterior ocular condition caused by or influenced by an ocular laser treatment; posterior ocular conditions caused by or influenced by a photodynamic therapy; photocoagulation; radiation retinopathy; epiretinal membrane disorders; branch retinal vein occlusion; anterior ischemic optic neuropathy; non-retinopathy diabetic retinal dysfunction, retinitis pigmentosa and glaucoma.

Glaucoma can also be considered a posterior ocular condition because a therapeutic goal of glaucoma treatment is to prevent the loss of or reduce the occurrence of loss of vision due to damage to or loss of retinal cells or optic nerve cells (i.e. neuroprotection).

The ocular disease or condition to be treated may be be wet (exudative) macular degeneration. "Treatment" and "therapy" may be used interchangeably. The therapy can be selected from, without limitation, initiating therapy, continuing therapy, modifying therapy or ending therapy. A therapy also includes any prophylactic measures that may be taken to prevent disease.

Treatment may comprise administering a disease-modulating drug to a subject. The drug can be a therapeutic or prophylactic used in subjects diagnosed or identified with a disease or at risk of having the disease. Modifying therapy refers to altering the duration, frequency or intensity of therapy, for example, altering dosage levels.

An "effective amount" or "therapeutically effective amount" refers to the amount of a compound or drug necessary to prevent, amerliorate or treat a condition or disease. In the case of age-related macular degeneration (AMD), the effective amount of the drug can, for example, be the amount necessary to reduce or prevent vision loss.

For AMD therapy, efficacy *in vivo* can, for example, be measured by one or more of the following: assessing the mean change in the best corrected visual acuity (BCVA) from baseline to a desired time, assessing the proportion of subjects who lose fewer than 15 letters in visual acuity at a desired time compared with baseline, assessing the proportion of subjects who gain greater than or equal to 15 letters in visual acuity at a desired time compared with baseline, assessing the proportion of subjects with a visual-acuity Snellen equivalent of 20/2000 or worse at desired time, assessing the National Eye Institute Visual Functioning Questionnaire, assessing the size of CNV and amount of leakage of CNV at a desired time, as assessed by fluorescein angiography.

"Therapeutic dose" refers is a dose which exhibits a therapeutic effect on the patient and a sub-therapeutic dose is a dose which does not exhibit a therapeutic effect on the patient treated.

### Epithelial Membrane Protein (EMP) 2

Epithelial membrane protein 2 (EMP2)(polypeptide; SEQ ID NO: 1)(nucleic acid; SEQ ID NO: 2) is a member of the growth arrest-specific gene 3/peripheral myelin protein 22 four-transmembrane protein family with distinctive biochemical and physiologic roles. EMP2 appears to regulate trafficking of various proteins and glycolipids by facilitating transfer of molecules from post-Golgi endosomal compartments to appropriate plasma membrane locations.

Specifically, EMP2 is thought to facilitate the appropriate trafficking of select molecules into glycolipid-enriched lipid raft microdomains. Glycolipid-enriched microdomains are cholesterol-rich microdomains, which are often associated with chaperones, receptosomes, and protein complexes that are important for efficient signal transduction.

Moreover, glycolipid-enriched microdomains are involved in correct sorting of proteins from the Golgi apparatus to plasma membrane. In this respect, modulation of EMP2 expression levels or its location on the plasma membrane alters the surface repertoire of several classes of molecules including integrins, focal adhesion kinase (FAK), class I MHC molecules, and other immunoglobulin superfamily members such as CD54 and glycosylphosphatidylinositol-linked proteins.

As described above, in the eye EMP2 is found in the cornea, ciliary boding, and RPE.

### Vascular Endothelial Growth Factor(VEGF)

VEGF is expressed in a variety of cells in the normal human retina. Co-localization of VEGF mRNA and protein is observed in the ganglion cell, inner nuclear and outer plexiform layers, the walls of the blood vessels, and photoreceptors (Gerhardinger et al., Am J Pathol 152:1453-62 (1998)). Retinal pigment epithelium, Muller cells, pericytes, vascular endothelium, and ganglion cells all produce VEGF (Miller et al., Diabetes Metab Rev 13:37-50 (1997); and, Kim et al. Invest Opthalmol Vis Sci 40:2115-21 (1999)).

Immunohistochemical studies have localized VEGF in surgically resected CNV membranes from AMD patients. Kvanta et al. (1996) described the presence of VEGF mRNA and protein in RPE cells and fibroblast like cells. *See* Kvanta et al., Invest Opthalmol Vis Sci 37:1929-34 (1996). Lopez et al. (1996) noted that the RPE cells that were strongly immunoreactive for VEGF were present primarily in the highly vascularized regions of CNV membranes, whereas the RPE cells found in fibrotic regions of CNV membranes showed little VEGF reactivity. See Lopez et al., Invest Opthalmol V is Sci 37:855-68 (1996). Kliffen et al. (1997) described increased VEGF expression in RPE cells and choroidal blood vessels in maculae from patients with wet AMD compared with controls. See Kliffen et al., Br J Opthalmol 81:154-62 (1997).

An increase in VEGF expression has been noted in experimental models of CNV in rats and in non human primates (Husain et al., Opthalmology 104:124250 (1997); and, Yi et al. Vascular endothelial growth factor expression in choroidal neovascularization in rats. Graefes Arch Clin Exp Opthalmol 235:313-9 (1997)). In addition, transgenic mice with increased VEGF expression in photoreceptors (Okamoto et al. 1997, supra) or retinal pigment epithelium (Schwesinger et al., AM J Pathol. 158(3):1161-72 (2001)) developed neovacularization reminiscent of CNV seen in humans with neovascular AMD.

Of interest to wet AMD are the angiogenic properties of VEGF, which are described in a variety of *in vivo* models, including the chick chorioallantoic membrane (Leung et al., Science 246:1306-9 (1989); and, Plouet J, Schilling J, Gospodarowicz D. EMBO J 8:3801-6 (1989)), rabbit cornea (Phillips et al., In Vivo 8:961-5 (1994)), and rabbit bone (Connolly et al. J Clin Invest 84:1470-8 (1989a)). VEGF also potentially functions as a survival factor for newly formed endothelial cells (Dvorak H F. N Engl J Med 315:1650-9 (1986); and, Connolly et al. J Biol Chem 264:20017-24 (1989)). Consistent with pro survival activity, VEGF induces expression of the anti apoptotic proteins Bel 2 and A1 in human endothelial cells (Connolly et al. J Biol Chem 264:20017-24 (1989b)).

VEGF has been shown to induce vascular leakage in guinea pig skin. A function of VEGF in the angiogenic process could be the induction of plasma protein leakage. This effect would result in the formation of an extravascular fibrin gel, which serves as a substrate for endothelial cells. Permeability of the CNV membranes could result in transudation of serum components beneath and into the retina, leading to serous macular detachment, macular edema and vision loss.

It has been shown that EMP2 regulates Focal Adhesion Kinase (FAK). FAK signal transduction is implicated in VEGF production.

### Pharmaceutical Compositions

Therapeutic compounds with optional pharmaceutically acceptable carriers, excipients or stabilizers (Remington's Pharmaceutical Sciences 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. An intraocular implant can be used for providing the EMP2 inhibitor. Examples of sustained-release preparations include semipermeable matrices of solid hydrophobic polymers containing a polypeptide of the invention, which matrices are in the form of shaped articles, e.g. films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and .gamma. ethyl-L-glutamate, nondegradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

Suspensions of microspheres (incorporating an agent) suspended in a hydrogel (such as a polymeric hyaluronic acid) can be administered to an intraocular location through a syringe needle.

The drug delivery systems of the invention can include a therapeutic agent mixed with or dispersed within a biodegradable polymer. The drug delivery systems compositions can vary according to the preferred drug release profile, the particular active agent used, the ocular condition being treated, and the medical history of the patient.

An intraocular drug delivery system can be made of a biodegradable polymer such as a poly(lactide) (PLA) polymers, poly(lactide-co-glycolide) (PLGA) polymers, as well as copolymers of PLA and PLGA polymers. PLA and PLGA polymers degrade by hydrolysis, and the degradation products, lactic acid and glycolic acid, are metabolized into carbon dioxide and water.

When encapsulated antibodies remain in the body for a long time, they may denature or aggregate as a result of exposure to moisture at 37°C., resulting in a loss of biological activity and possible changes in immunogenicity.

Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions.

### Administrative modalities

The therapeutic compound for treatment of an intraocular neovascular disease is typically administered by ocular, intraocular, and/or intravitreal injection. Other methods administration can also be used, which includes but is not limited to, topical, parenteral, for example, subcutaneous, intraperitoneal, intrapulmonary, intranasal, and intralesional administration.

Parenteral infusions include intramuscular, intravenous, intraarterial, intraperitoneal, or subcutaneous administration. As described herein, the therapeutic compound for treatment of an intraocular neovascular syndrome may be formulated, dosed, and administered in a fashion consistent with good medical practice.

"Intraocular" refers to within or under an ocular tissue. An intraocular administration of a drug delivery system includes administration of the drug delivery system to a sub-Tenon, subconjunctival, suprachoroidal, intravitreal and like locations. An intraocular administration of a drug delivery system excludes administration of the drug delivery system to a topical, systemic, intramuscular, subcutaneous, intraperitoneal, and the like location.

A "therapeutic agent," "active agent," and "drug" are used interchangeably and refer to any substance (including a biologic or macromolecule) used to treat an ocular condition. A therapeutic agent can be administered to the eye to treat an ocular condition. It is known to administer a drug depot to the posterior (i.e. near the macula) sub-Tenon space. *See* eg column 4 of U.S. Pat. No. 6,413,245. Additionally, it is known to administer a polylactic implant to the sub-tenon space or to a suprachoroidal location. See eg published U.S. Pat. No. 5,264,188 and published U.S. patent application 20050244463

An anti-EMP2 agent can be used for the treatment of an ocular condition, such as a posterior ocular condition, which involves angiogenesis such as choroidal neovascularization ("CNV"). A therapeutic amount of an anti-EMP2 agent can be administered to the eye directly, for example into the vitreous. Maurice, D. M. (1983) Micropharmaceutics of the eye, Ocular Inflammation Ther. 1:97-102; Lee, V. H. L. et al. (1989), Drug delivery to the posterior segment" Chapter 25 In Retina. T. E. Ogden and A. P. Schachat eds., St. Louis: C V Mosby, Vol. 1, pp. 483-98; and Olsen, T. W. et al. (1995), Human scleral permeablilty: effects of age, cryotherapy, transscleral diode laser, and surgical thinning, Invest. Opthalmol. Vis. Sci. 36:1893-1903.

Intraocular administration can be injected to an intraocular location by syringe or can be inserted (implanted) into the eye by a variety of methods, including placement by forceps, by trocar, or by other types of applicators, after making an incision in the sclera.

In some instances, a trocar or applicator may be used without creating an incision. A hand held applicator may be used to insert one or more biodegradable implants into the eye. The hand held applicator typically comprises an 18-30 GA stainless steel needle, a lever, an actuator, and a plunger. Suitable devices for inserting an implant or implants into a posterior ocular region or site includes those disclosed in U.S. patent application publication number US 2005-0101967 A1.

The method of administration generally first involves accessing the target area within the ocular region with the needle, trocar or implantation device. Once within the target area, e.g., the vitreous cavity, a lever on a hand held device can be depressed to cause an actuator to drive a plunger forward. As the plunger moves forward, it can push the implant or implants into the target area (i.e. the vitreous by implantation or injection into the vitreous cavity (posterior chamber) of the eye.

The drug delivery systems can be biodegradable implants and/or microspheres. The drug delivery systems can be monolithic, that is the active agent is homogenously distributed or dispersed throughout the biodegradable polymer.

EP 488 401 discusses intraocular implants, made of certain polylactic acids, to be applied to the interior of the eye after a surgical operation for disorders of the retina/vitreous body. EP 430539 discusses use of a bioerodible implant which is inserted in the suprachoroid. U.S. application publication number US 2008-0131484 A1 filed Dec. 1, 2006 discloses intraocular (including sub-tenon's) administration of various solid, drug-containing implants.

Intraocular drug delivery systems which are sutured or fixed in place are known. Suturing or other fixation means requires sensitive ocular tissues to be in contact with aspects of a drug delivery system which are not required in order to contain a therapeutic agent within or on the drug delivery system or to permit the therapeutic agent to be released in vivo. As such suturing or eye fixation means a merely peripheral or ancillary value and their use can increase healing time, patient discomfort and the risk of infection or other complications.

U.S. patent applications publication numbers US 2008-0268051 A1 ; US 2009-0081277 A1 ; US 2009-0148527 A1 ; US 2007-0059336 A1 discuss use of intraocular compositions comprising therapeutic antibodies, such as bevacizumab. Formulations of macromolecules for intraocular use are known, See eg applications publication numbers US 2006-0182783 A1 ; US 2007-0059336 A1 ; US 2005-0281861 A1 and Ser Nos.60/567,423 and 60/721,600 abandoned before publication.

### Treatment modalities

The doses may be administered according to any time schedule which is appropriate for treatment of the disease or condition. For example, the dosages may be administered on a daily, weekly, biweekly or monthly basis in order to achieve the desired therapeutic effect and reduction in adverse effects.

The dosages can be administered before, during or after the development of the disorder. The specific time schedule can be readily determined by a physician having ordinary skill in administering the therapeutic compound by routine adjustments of the dosing schedule within the method of the present invention.

The time of administration of the number of first individual and second individual doses as well as subsequent dosages is adjusted to minimize adverse effects while maintaining a maximum therapeutic effect. The occurrence of adverse effects can be monitored by routine patient interviews and adjusted to minimize the occurrence of side effects by adjusting the time of the dosing. Any dosing time is to be considered to be within the scope of the present invention. For example, doses may be administered on a monthly schedule followed by subsequent quarterly or more dose schedule. Maintenance doses are also contemplated by the invention.

The dosage amount depends on the specific disease or condition which is treated and can be readily determined using known dosage adjustment techniques by a physician having ordinary skill in treatment of the disease or condition. The dosage amount will generally lie with an established therapeutic window for the therapeutic compound which will provide a therapeutic effect while minimizing additional morbidity and mortality. Typically, therapeutic compounds are administered in a dosage ranging from 0.001 mg to about 100 mg per dose, preferably 0.1-20 mg.

Typically, the therapeutic compound used in the methods of this invention is formulated by mixing it at ambient temperature at the appropriate pH, and at the desired degree of purity, with physiologically acceptable carriers, i.e., carriers that are non-toxic to recipients at the dosages and concentrations employed. The pH of the formulation depends mainly on the particular use and the concentration of antagonist, but preferably ranges anywhere from about 3 to about 8.

The therapeutic compound, for example an anti-EMP2 antibody, for use herein is sterile. Sterility can be readily accomplished by sterile filtration through (0.2 micron) membranes. Preferably, therapeutic peptides and proteins are stored as aqueous solutions, although lyophilized formulations for reconstitution are acceptable.

The therapeutic compound may be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the time scheduling of administration, and other factors known to medical practitioners.

It is also to be understood that the terminology used herein is for the purpose of describing particular embodiments only, and is not intended to be limiting. As used in this specification and the appended claims, the singular forms "a", "an" and "the" include plural referents unless the content clearly dictates otherwise. Thus, for example, reference to "a molecule" optionally includes a combination of two or more such molecules, and the like. The conjunction "or" is not mutually exclusive, unless context clearly dictates otherwise. The term "include" is used to refer to non-limiting examples.

### EXAMPLES

The following examples are offered to illustrate, but not to limit the invention.

### EXAMPLE 1: ARPE-19 Cell Line

ARPE-19, a spontaeously arising retinal pigment epithelial (RPE) cell line that expresses the RPE-specific markers CRALBP and RPE-65, was obtained from the American Type Culture Colletion (CRL-2302; ATCC™). ARPE-19 cells were cultured in DMEM-F12 medium, supplemented with 10% fetal bovine serum at 37°C in a humidified chamber with 5% CO₂. The culture medium was replaced twice a week. After confluence, the cultures were passaged by dissociation in 0.05% (wt/vol) trypsin. Levels of EMP2 were increased

### EXAMPLE 2: Phage Library Selection

10¹² to 10¹³ phage from the 8.2 × 10⁸ member phagemid library were first predepleted with 100 µL streptavidin magnetic beads (Invitrogen) in 2% milk PBS for 1 h at room temperature. The predepleted phage library was then mixed with 10 µg biotin-conjugated 24-amino acid peptides corresponding to the extracellular loop of hEMP2 and mouse EMP2 (mEMP2; DIHDKNAKFYPVTREGSYGGSGSK and DLHQQNRKLYYLLQEGSYGGSGSK, respectively; ref. 27) for 1 h at room temperature. One hundred microliters of 2% milk PBS preblocked streptavidin magnetic beads were added to the phage mixture and rotated for 15 min at room temperature. Beads were washed extensively with 0.1% PBS/Tween 20, 2% milk PBS, and finally with PBS, and bound phage was eluted out with 1 mL of 100 mmol/L triethylamine, neutralized with 500 µL of 1 mol/L Tris-HCl (pH 7.4), and added to 10 mL exponentially growing Escherichia coli TG1. Culture was then plated on 150 mm culture plates with 2× TY 100 µg/mL ampicillin and 2% glucose agar plates overnight at 37°C. The next day, colonies were scraped from the plates and used to amplify the phage for the second round of selection described above. A total of three selections were done before screening and characterization of the selected phage antibodies.

### EXAMPLE 3: Diabody construction and production

Binding specificity of expressed scFv was analyzed by ELISA. scFv clones with high reactivity were selected for the construction of diabodies. Several different scFv clones were characterized and confirmed by DNA fingerprinting and DNA sequencing. pHEN phagemids from selected phage were isolated using QIAprep Spin Miniprep Kit (Qiagen). scFv inserts were then digested and cloned into pSYN I vector in-frame with a c-Myc and 6 His tag at the COOH terminus. To convert scFv fragments into diabody, 15-amino acid linker region (AGTGGTGGAGGCGGTTCAGGCGGAGGTGGCTCTGGCGGTGGCGGATCG) of the scFv was shortened into 5-amino acid linker (AGTGGTGGAGGATCG) using QuikChange site-directed mutagenesis kit (Stratagene). Deletion mutation was confirmed by DNA sequencing analysis.

Expression and purification of the selected diabodies were carried out using a modified protocol described by Marks and Bradbury. Single colonies were picked from the plate and inoculated into 1 L/colony of 2× TY with 100 µg/mL ampicillin at 250 rpm at 37°C. When *A*₆₀₀ reached 0.8 to 1.0, protein expression was induced by addition of 1 mmol/L IPTG. The culture was shaken at 120 rpm at 30°C for 4 h and spun at 7,000 rpm for 15 min at 4°C. Pellets were then resuspended in 20 mL periplasmic buffer [200 mmol/L Tris-HCl, 20% sucrose, 1 mmol/L EDTA (pH 7.5)], and 290,000 units lysozyme (Epicentre) was added to each mixture. The mixtures were incubated at room temperature for 5 min and spun at 7,000 rpm for 15 min at 4°C. The pellets were then resuspended with 20 mL of 40 mmol/L MgSO₄ and left on the ice for 10 min. The samples were spun again, and the supernatants from this spin were combined with the first supernatants. The mixture was then filtered with 0.45 µm filters and dialyzed in dialysis buffer [300 mmol/L NaCl, 20 mmol/L HEPES (pH 8.0)] overnight at 4°C. Next morning, the samples were filtered again with 0.2 µm filters and run through 5 mL of the Ni-NTA column (Qiagen). The column was washed with 20 mL wash buffer [300 mmol/L NaCl, 20 mmol/L imidazole, 20 mmol/L HEPES, 0.05% Tween 20 (pH 8.0)], and bound diabodies were eluted with 5 mL elusion buffer [300 mmol/L NaCl, 250 mmol/L imidazole, 20 mmol/L HEPES (pH 8.0)] and dialyzed in endotoxin-free PBS overnight at room temperature. Samples were filtered with 0.22 µm filters and stored at -20°C until their use. Purity of the preparation was determined by size-exclusion chromatography profile (fast protein liquid chromatography; Superdex 75™; Amersham Pharmacia Biotech) as necessary.

For preparative analysis of the diabody, purified diabody preparations were run on 4% to 20% Tris-glycine gel (Invitrogen) and bands were visualized using GelCode™ Blue Stain Reagent (Pierce). Gels were scanned and the band intensities were analyzed using the Image J program (NIH).

### EXAMPLE 4: VEGF ELISA

The concentration of VEGF was determined using VEGF ELISA kit (R & D Systems, Minneapolis, MN) following the manufacturer's instructions.

### EXAMPLE 5: siRNA EMP2

EMP2 levels were decreased by transiently transfecting ARPE-19 cells with 75 picomoles EMP2 siRNA (J-016226-05 (SEQ ID NO:3), J-016226-06 (SEQ ID NO: 4), J-016226-07 (SEQ ID NO:5), J-016226-08 (SEQ ID NO:6); target sequecnces; Dharmacon, Lafayette, CO) and lipophilic transfection reagent (Lipofectamine™ 2000; Invitrogen, Carlsbad, CA) and analyzed after 48 hours. As a negative control, the cells were transfected with 75 picomoles of scramble control siRNA (D-001206-13-05; Dharmacon). The EMP2 siRNA and control siRNA are a pool of four siRNAs targeting EMP2 or a pool of four nontargeting siRNAs, respectively. The level of VEGF expression was quantified by Western blot and ELISA as described.

### EXAMPLE 6: VEGF Western Blot Analysis

Western blot analysis was performed as previously described. Briefly, cell protein was isolated by using RIPA buffer containing protease and phosphatase inhibitors (Upstate, Charlottesville, VA) and the protein concentration determined with a protein assay (bicinchoninic acid (BCA); Bio-Rad). A total of 10 µg protein was loaded in each lane and the proteins fractionated by 4% to 20% SDS-PAGE gradient gel in reducing conditions. Proteins were transferred to nitrocellulose membranes (GE Healthcare, Buckinghamshire, UK) and the adequacy of transfer confirmed by Ponceau S red staining (Sigma-Aldrich). The membrane was then blocked with nonfat milk in TBS Tween (TBST; Upstate). Blots were incubated for 1 hour with primary antibody at a dilution of 1: for ant-VEGF and 1:5000 for β-actin. Horseradish peroxidase-conjugated goat anti-rabbit or horseradish peroxidase-conjugated goat anti-mouse was exposed to the blots at a 1:2000 dilution. The blots were then developed with chemiluminescence to visualize bound antibody (ECL; Pierce, Rockford, IL) and quantified with β-actin as the internal control. The Western blot analyses were quantified with NIH Image J. The blots were digitized with a flatbed scanner, and the band density was measured by using Image J. To account for loading variability, β-actin was used to normalize each sample. At least three independent experiments were performed and, where indicated, the results were evaluated for statistical significance with a Student's *t*-test (unpaired, one-tail). A level of *P*< 0.05 was considered to be statistically significant.
SEQ ID NO:1 Epithelial Membrane Protein 2 (NP_001415.1) 
MLVLLAFIIAFHITSAALLFIATVDNAWWVGDEFFADVWRICTNNTNCTVINDSF
QEYSTLQAVQATMILSTILCCIAFFIFVLQLFRLKQGERFVLTSIIQLMSCLCVMIA
ASIYTDRREDIHDKNAKFYPVTREGSYGYSYILAWVAFACTFISGMMYLILRKRK
SEQ ID NO:2 Epithelial Membrane Protein 2 (NM_001424) 
GAGGGGCCCC GCCGCCTAGA GGGTGGAGGG AGGGCGCGCA GTCCCAGCCC
AGAGCTTCAA AACAGCCCGG CGGCCTCGCC TCGCACCCCC AGCCAGTCCG
TCGATCCAGC TGCCAGCGCA GCCGCCAGCG CCGGCACATC CCGCTCTGGG
CTTTAAACGT GACCCCTCGC CTCGACTCGC CCTGCCCTGT GAAAATGTTG
GTGCTTCTTG CTTTCATCAT CGCCTTCCAC ATCACCTCTG CAGCCTTGCT
GTTCATTGCC ACCGTCGACA ATGCCTGGTG GGTAGGAGAT GAGTTTTTTG
CAGATGTCTG GAGAATATGT ACCAACAACA CGAATTGCAC AGTCATCAAT
GACAGCTTTC AAGAGTACTC CACGCTGCAG GCGGTCCAGG CCACCATGAT
CCTCTCCACC ATTCTCTGCT GCATCGCCTT CTTCATCTTC GTGCTCCAGC
TCTTCCGCCT GAAGCAGGGA GAGAGGTTTG TCCTAACCTC CATCATCCAG
CTAATGTCAT GTCTGTGTGT CATGATTGCG GCCTCCATTT ATACAGACAG
GCGTGAAGAC ATTCACGACA AAAACGCGAA ATTCTATCCC GTGACCAGAG
AAGGCAGCTA CGGCTACTCC TACATCCTGG CGTGGGTGGC CTTCGCCTGC
ACCTTCATCA GCGGCATGAT GTACCTGATA CTGAGGAAGC GCAAATAGAG
TTCCGGAGCT GGGTTGCTTC TGCTGCAGTA CAGAATCCAC ATTCAGATAA
CCATTTTGTA TATAATCATT ATTTTTTGAG GTTTTTCTAG CAAACGTATT
GTTTCCTTTA AAAGCCAAAA AAAAAAAAAA AAAAAAAAAA
AAAAGAAAAA AGAAAAAAAA AATCCAAAAG AGAGAAGAGT TTTTGCATTC
TTGAGATCAG AGAATAGACT ATGAAGGCTG GTATTCAGAA CTGCTGCCCA
CTCAAAAGTC TCAACAAGAC ACAAGCAAAA ATCCAGCAAT GCTCAAATCC
AAAAGCACTC GGCAGGACAT TTCTTAACCA TGGGGCTGTG ATGGGAGGAG
 
AGGAGAGGCT GGGAAAGCCG GGTCTCTGGG GACGTGCTTC CTATGGGTTT
CAGCTGGCCC AAGCCCCTCC CGAATCTCTC TGCTAGTGGT GGGTGGAAGA
GGGTGAGGTG GGGTATAGGA GAAGAATGAC AGCTTCCTGA GAGGTTTCAC
CCAAGTTCCA AGTGAGAAGC AGGTGTAGTC CCTGGCATTC TGTCTGTATC
CAAACCAGAG CCCAGCCATC CCTCCGGTAT CGGGGTGGGT CAGAAAAAGT
CTCACCTCAA TTTGCCGACA GTGTCACCTG CTTGCCTTAG GAATGGTCAT
CCTTAACCTG CGTGCCAGAT TTAGACTCGT CTTTAGGCAA AACCTACAGC
GCCCCCCCCC TCACCCCAGA CCTACAGAAT CAGAGTCTTC AAGGGATGGG
GCCAGGGAAT CTGCATTTCT AACGCGCTCC CTGGGCAACG CTTCAGATGC
GTTGAAGTTG GGGACCACGG TGCCTGGGCC AGGTCAGCAG AGCTGCCTCG
TAAATGCTGG GGTATCGTCA TGTGGAGATG GGGAGGTGAA TGCAACCCCC
ACAGCAGGCC AAAACCTTGG CCTCCATCGC CACAGCTGTC TACATCTAGG
GCCCCAAAAC TCCATTCCTG AGCCATGTGA ACTCATAGAC ACCTTCAGGG
TGTGGGGTAC AGCCTCCTTC CCATCTTATC CCAGAAGGCC TCTCCCTTCT
TGTCCAGCCC TTCATGCTAC ACCTGGCTGG CCTCTCACCC CTATTTCTAG
AGCCTCAGAG GACCCATCCA CCATTCATTC ATTCATTCAT TCATTCATTC
ATTCATTCAT TCATCAACAT AAATCATAAC TTGCATGCAT GTGCCAGGCA
CAGGGGATAC CCTCTAGAGA CAATCTCCTC CTAGGGCTCA TGGCCTAGTG
GAGGAGACAG ATTAAAACTT AATTAGAAAA ACTGGCTGGG TACAGTGGCT
CATGCTTGTA ATCCCAGCAC TTTGGGAGGC TGAGGCGGGT GGATCACCTG
AGGTCAGGAG TTCAAGACCA GCCTGGCCAA AATGGTAAAA CCTGTCTCTA
CTAAAAATAC AAAAATGAGC TGGGCGTGGT GGTGCATGCC TGTAATCCCA
GCTATCAGGT GGCTGAGGCA GGAGAATCAC TTGAAATGGG AGGTGGAGGT
TGCAGTGAGC CGAGACCGTG CCACTGCACT CCAGCCTGGG TGACAGAGTG
AGACTCCATC TCAAAAAAAG AAAAAAAAGA AAAGAAACTA ATTACACACT
GTGATGGAGG CTGCAAAGAA CACCACTAAG AATTCAAAAT CAGCTGGGTG
CGGTGGCTCA CACCTGTAAT CCCAGCACTT TGGGAGGCTG AGGCAGGTGG
ATCACAAGGT CAGGAGTTCA AGACCAGCCT GGCCAACATG GTGAAACCCC
GTCTCTACCG AAAATACAAC AAAATTAGCC CGGTGTGGTG GCAGGTGCCT
GTAATCCCAG CTACTTAGGA GGCTGAGGCA GGAGAATCGC TTGAAACTGG
GAGGCGGAGG TCGCAGTGAG CCGAGATTCA CCACTGCACT CCAGCCCAGG
CGACAGTCTG AGACTCCGTC TCAAAAATAA AACGATTCAA AATCGAGGCC
TGTGGCATGG TAGGGAGGCT GCTTTACGCG TGCCTATTAT TAAATGCTCC
TGGAGGCATT TAGGTATTTA GATCAGTCTA AATATAGCTC CATTCAGTTC
GTGCAGATGA CAGTTATTGG GCAGTACCTG TCTGTGTAAC ACCCAGAAAA
CATGTCTGTG GAGGGGCCCA TGGTCCCGAC AGTAAATGCG GTGAGAGGGT
 
CCCATAGAGC TGGAGTTTTC AAGCTTTAGG GGTTCCCGTG CTGCTTGGGA
CAGGCTGATT CAGAGGGTCT GGGTGAATGA TTTCCAGGTG ATTTTAAGAC
TGTGCTGAGA AATAGGGCTT TTGGGGCCTT GTCCTTCAGG ATCAAAGCAT
GATGCTGTGT GGCAATGCAG ACCACCCAGG AACCATCCCA GGAGATAAGC
TCTTTGCACC TCATTGTCTT TTTCTGCTTA TGTTGGAGCA GGATGCTGGG
GGCTGTCCTG GGATGGGGTG TGGGACCTCG TGCTATTTAA ATACTTTTGC
ACTTGACCTT CTGCTGAGTG GAGTGGTGGT TTGCCATCAG CTCAGTTCCA
GTGGAGCTGA AGAGACATCT GGTTTGAGTA GTTTTAGGGC CACCATGGAT
ATCTCTTCAA TGCAGGATTG GCTCTTTCCA TCTGCTCTTT CATTCATTTG
TTTTTGACAG ATAGTATTAA ATGTTTACCA TGTTCCAGGC ACTGTGTGAG
GCTCTGAAAA TACAGGGGTG AGCAAATCCA GATATCCTCC CTGCCATCAT
GAAGTTTGGA GTCTATGAGA TAGGACCCCC TCCCTATGGA GAAGCCACCA
ATGCAGTACA GGGTGACCTG GGGCCAGAGA CAGGACAAAT GTCACCTCCT
GCCTCCATGA GATACTCTCA CTAGTCATAT TGTGGGCAAG AATGTGGCTT
ACACCCCTAG GGTTAACAGG ATGCTACCCA AGCTCATGGA GGAAGTTGAA
TCTTAAGTTC CCTTGAAACT TTCTACCTTG GTGGCTTTTC TATAATTTTC
TTTTTTCTTT TTCTTTTTTT TTTTTTTTTT TGAGACTGAG TTTTGCTCTT
GTTGCCCAGG CTGGAGTGCA GTGGCACCAT CTTGGCTCAC CGCAACCTCT
GCCTCCTGGG TTCAAGTGAT TCTCCTGCCT CAGCCTCCCG AGTAGCTGGG
ATTACAGGCA TGTCCCACCA TGCCCAGCTA ATTTTTGTAT TTTTAGTAGA
GATGGGGTTT CTCCATGTTG GTCAGGCTGG TTTCGAACTC CCAACCTCAG
GTGATCCGCC CACCTCAGCC TTCCAAAGTG CTGGGATTAC AGGCATGAGC
CACTGCGTCT GGCCTTCTAT AATTTTCTGG TAGTCACGAT GGAAACAAAC
AAAACACCTT AGAACCAGAG ATCGACCCCC TCAAGCAATA CATCAATTCC
CTTCACAAGA AACGTCGGGG CTACATGAGT ATCTGTGTTG AATGCGGTCT
GAAATGATCC TATGGATTTT CCCGGCTGGT TGCCACTGCT GTACAACATT
CAGTGCCCAC ATCCACCTGT GCCATTAAGC TTTTTTGAGA CATGAGAGAT
GCCTCTTCCC TGCTGTATGA CATGCATTTG GGAAGTTGGA AAGAAATGAC
AAAATCAGGG AGAAAACATC CAAGCTTCTT ACCTGTAGAT AGAATCAGCC
CTCACTTGGT GCTTATTACC AGTTATTCAA GAACAATAAC AACAACAAAA
TTAGTAGACA TCCAAGAAGC ACATATTAGG ACCAAAGATA GCATCAACTG
TATTTGAAGG AACTGTAGTT TGCGCATTTT ATGACATTTT TATAAAGTAC
TGTAATTCTT TCATTGAGGG GCTATGTGAT GGAGACAGAC TAACTCATTT
TGTTATTTGC ATTAAAATTA TTTTGGGTCT CTGTTCAAAT GAGTTTGGAG
AATGCTTGAC TTGTTGGTCT GTGTGAATGT GTATATATAT ATACCTGAAT
ACAGGAACAT CGGAGACCTA TTCACTCCCA CACACTCTGC TATAGTTTGC
 
GTGCTTTTGT GGACACCCCT CATGAACAGG CTGGCGCTCT AGGACGCTCT
GTGTTCACTG ATGATGAAGA AACCTAGAAC TCCAAGCCTG TTTGTAAACA
CACTAAACAC AGTGGCCTAG ATAGAAACTG TATCGTAGTT TAAAATCTGC
CTCGCGGGAT GTTACTAAAC TCGCTAATAG TTTAAAGGTT ACTTACAATA
GAGCAAGTTG GACAATTTTG TGGTGTTGGG GAAATGTTAG GGCAAGGCCT
AGAGGTTCAT TTTGAATCTT GGTTTGTGAC TTTAGGGTAG TTAGAAACTT
TCTACTTAAT GTACCTTTAA AATAGTCCAT TTTCTATGTT TTGTATAATC
TGAAACTGTA CATGGAAAAT AAAGTTTAAA ACCAGATTGC CCAGAGCAAG
ACTCTAATGT TCCCAACGGT GATGACATCT AGGGCAGAAT GCTGCCATTT
TGAGGGGCAG GGGGTCAGCT GATTTCTCAT CAAGATAATA ATGTATGGTT
TTTACACTAA GCAACTGATA AATGGACAAT TTATCACTGG AAAAAA
SEQ ID NO:3 (Target Sequence 1): GUACCAACAACACGAAUUG
SEQ ID NO:4 (Target Sequence 2): UGAUUGCGGCCUCCAUUUA
SEQ ID NO:5 (Target Sequence 3): GCGAAAUUCUAUCCCGUGA
SEQ ID NO:6 (Target Sequence 4): AGACAGGCGUGAAGACAUU
SEQ ID NO:7 KS49 heavy chain 
MAQVQLVQSGGGVVQPGRSLRLSCAASGFTFSSYAMHWVRQAPG
KGLEWVAVISYDGSNKYYADSVKGRFTISRDNSKNTLYLQMNSLR
AEDTAVYYCARDRRGRKSAGIDYWGQGTLVTVSS
SEQ ID NO:8 KS49 heavy chain CDR1: SYAMH
SEQ ID NO:9 KS49 heavy chain CDR2: VISYDGSNKYYADSVKG
SEQ ID NO:10 KS49 heavy chain CDR3: DRRGRKSAGIDY
SEQ ID NO:11 KS49 light chain 
DIQMTQSPSSLSASVGDRVTITCQASQDISNYLNWYQQKPGKAPK
LLIYAASSLQSGVPSRFSGSGSGTDFTLTISSLQPEDFATYYCLQDY
NGWTFGQGTKVDIKRAAAEQKLISEEDLNGAA
SEQ ID NO:12 KS49 light chain CDR1: QASQDISNYLN
SEQ ID NO:13 KS49 light chain CDR2: AASSLQS
SEQ ID NO:14 KS49light chain CDR3: LQDYNGWT
SEQ ID NO:15 KS83 heavy chain 
MAQVQLVESGGGLVQPGGSLRLSCAASGFTFSSYAMHWVRQAPG
KGLEWVAVISYDGSNKYYADSVKGRFTISRDNSKNTLYLQMNSLR
AEDTAVYYCARTVGATGAFDIWGQGTMVTVSSS
SEQ ID NO:16 KS83 heavy chain CDR1: SYAMH
SEQ ID NO:17 KS83 heavy chain CDR2: VISYDGSNKYYADSVKG
SEQ ID NO:18 KS83 heavy chain CDR3: TVGATGAFDI
SEQ ID NO:19 KS83 light chain-DIVMTQSPSTVSASVGD RV IIPCRASQSIGKWLAWYQQKPGKAPKLLIYKASSLEGWVPSRFSGSGSGTEFSLTI SSLQPDDSATYVCQQSH NFPPTFGGGTKLEIKRAAAEQKLISEEDLNGAA
SEQ ID NO:20 KS83 light chain CDR1: RASQSIGKWLA
SEQ ID NO:21 KS83 light chain CDR2: KASSLEG
SEQ ID NO:22 KS83 light chain CDR3: QQSHNFPPT
SEQ ID NO:23 KS41 Heavy Chain- 
MAQVQLVQSGGGLVQPGRSLRLSCAASGFSFSEYPMHWVR
QAPGRGLESVAVISYDGEYQKYADSVKGRFTISRDDSKSTV
YLQMNSLRPEDTAVYYCARTINNGMDVWGQGTTVTVSS
SEQ ID NO:24 KS41 heavy chain CDR1: EYPMH
SEQ ID NO:25 KS41 heavy chain CDR2: VISYDGEYQKYADSVKG
SEQ ID NO:26 KS41 heavy chain CDR3: TINNGMDV
SEQ ID NO:27 KS41 Light Chain- 
DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA
SEQ ID NO:28 KS41 light chain CDR1: RASQGIRNDLG
SEQ ID NO:29 KS41 light chain CDR2: GASSLQS
SEQ ID NO:30 KS41 light chain CDR3: LQDYNGWT
SEQ ID NO:31 KS89 Heavy Chain- 
MAQVQLVQSGGGLVQPGRSLRLSCAASGFSFSEYPMHWVRQAPGRGLESVAVIS
YDGEYQKYADSVKGRFTISRDDSKSTVYLQMNSLRPEDTAVYYCARTINNGMD
VWGQGTTVTVSS
SEQ ID NO:32 KS89 heavy chain CDR1: EYPMH
SEQ ID NO:33 KS89 heavy chain CDR2: VISYDGEYQKYADSVKG
SEQ ID NO:34 KS89 heavy chain CDR3: TINNGMDV
SEQ ID NO:35 KS89 Light Chain- 
DIVMTQSPSSLSASVGDRVTITCRASQGIRNDLGWYQQKPGKAPELLIYGASSLQ
SGVPSRFSGSGSGTDFTLTISSLQPEDSATYYCLQDYNGWTFGQGTKLEIKRAAA
EQKLISEEDLNGAA
SEQ ID NO:36 KS89 light chain CDR1: RASQGIRNDLG
SEQ ID NO:37 KS89 light chain CDR2: GASSLQS
SEQ ID NO:38 KS89 light chain CDR3: LQDYNGWT
SEQ ID NO: 39 KS49 full polypeptide sequence of anti-EMP-2 diabody 
MAQVQLVQSGGGVVQPGRSLRLSCAASGFTFSSYAMHWVRQAPG
KGLEWVAVISYDGSNKYYADSVKGRFTISRDNSKNTLYLQMNSLRAEDTAVYY
CARDRRGRKSAGIDYWGQGTLVTVSSGGGSDIQMTQSPSSLSASVGDRVTITCQ
ASQDISNYLNWYQQKPGKAPKLLIYAASSLQSGVPSRFSGSGSGTDFTLTISSLQP
EDFATYYCLQDY NGWTFGQGTKVDIKRAAAEQKLISEEDLNGAAHHHHHH
SEQ ID NO:40 KS83 full polypeptide sequence of anti-EMP-2 diabody 
MAQVQLVESGGGLVQPGGSLRLSCAASGFTFSSYAMHWVRQAPGKGLEWVAVI
SYDGSNKYYADSVKGRFTISRDNSKNTLYLQMNSLRAEDTAVYYCARTVGATG
AFDIWGQGTMVTVSSSGGGSDIVMTQSPSTVSASVGDRVIIPCRASQSIGKWLAW
YQQKPGKAPKLLIYKASSLEGWVPSRFSGSGSGTEFSLTISSLQPDDSATYVCQQS
HNFPPTFGGGTKLEIKRAAAEQKLISEEDLNGAAHHHHHH
SEQ ID NO:41 KS41 full polypeptide sequence of anti-EMP-2 diabody 
MAQVQLVQSGGGLVQPGRSLRLSCAASGFSFSEYPMHWVRQAPGRGLESVAVIS
YDGEYQKYADSVKGRFTISRDDSKSTVYLQMNSLRPEDTAVYYCARTINNGMD
VWGQGTTVTVSSSGGGSDIVMTQSPSSLSASVGDRVTITCRASQGIRNDLGWYQ
QKPGKAPELLIYGASSLQSGVPSRFSGSGSGTDFTLTISSLQPEDSATYYCLQDYN
GWTFGQGTKLEIKRAAAEQKLISEEDLNGAAHHHHHH
SEQ ID NO:42 KS89 full polypeptide sequence of anti-EMP-2 diabody

MAQVQLVQSGGGLVQPGRSLRLSCAASGFSFSEYPMHWVRQAPGRGLESVAVIS
YDGEYQKYADSVKGRFTISRDDSKSTVYLQMNSLRPEDTAVYYCARTINNGMD
VWGQGTTVTVSSSGGGSDIVMTQSPSSLSASVGDRVTITCRASQGIRNDLGWYQ
QKPGKAPELLIYGASSLQSGVPSRFSGSGSGTDFTLTISSLQPEDSATYYCLQDYN
GWTFGQGTKLEIKRAAAEQKLISEEDLNGAAHHHHHH

## Claims

1. An epithelial membrane protein 2 (EMP2) inhibitor for use in treating wet age related macular degeneration (AMD), wherein the EMP2 inhibitor is selected from a group consisting of an anti-EMP2 antibody, an EMP2 si-RNA, and combinations thereof.

2. An epithelial membrane protein 2 (EMP2) inhibitor for use according to claim 1, wherein the EMP2 inhibitor is an anti-EMP2 antibody.

3. An epithelial membrane protein 2 (EMP2) inhibitor for use according to claim 1, wherein the EMP2 inhibitor is an EMP2 si-RNA.

4. An epithelial membrane protein 2 (EMP2) inhibitor for use according to claim 1, wherein the EMP2 inhibitor comprises at least two epithelial membrane protein 2 (EMP2) inhibitors.

5. An epithelial membrane protein 2 (EMP2) inhibitor for use according to claim 4, wherein one of the at least two EMP2 inhibitors is an anti-EMP2 antibody and wherein one of the at least two EMP2 inhibitors is an EMP2 si-RNA.

6. An epithelial membrane protein 2 (EMP2) inhibitor for use in treating a disease of the eye, wherein:
the disease of the eye is diabetic retinopathy, corneal neovascularization, choroidal neovascularization, cyclitis, Hippel-Lindau Disease, retinopathy of prematurity, pterygium, histoplasmosis, iris neovascularization, macular edema, glaucoma-associated neovascularization, and Purtscher's retinopathy, and
the EMP2 inhibitor is selected from a group consisting of an anti-EMP2 antibody, an EMP2 si-RNA, and combinations thereof.

7. An epithelial membrane protein 2 (EMP2) inhibitor for use in reducing the expression of vascular endothelial growth factor (VEGF) in the eye of a patient suffering from a disease selected from the group consisting of wet age related macular degeneration (AMD), diabetic retinopathy, corneal neovascularization, choroidal neovascularization, cyclitis, Hippel-Lindau Disease, retinopathy of prematurity, pterygium, histoplasmosis, iris neovascularization, macular edema, glaucoma-associated neovascularization, and Purtscher's retinopathy,
wherein the EMP2 inhibitor is selected from a group consisting of an anti-EMP2 antibody, an EMP2 si-RNA, and combinations thereof.

8. An epithelial membrane protein 2 (EMP2) inhibitor for use according to claim 7, wherein the EMP2 inhibitor is an anti-EMP2 antibody.

9. An epithelial membrane protein 2 (EMP2) inhibitor for use according to claims 1, 2, 6, 7, or 8, wherein the anti-EMP2 antibody comprises a heavy chain and a light chain, wherein the heavy chain comprises three complementarity determining regions (CDRs): HCDR1, HCDR2, and HCDR3, and wherein the light chain comprises three CDRs: LCDR1, LCDR2, and LCDR3, wherein HCDR1 comprises SEQ ID NO:8, HCDR2 comprises SEQ ID N0:9, and HCDR3 comprises SEQ ID NO: 10, and wherein LCDR1 comprises SEQ ID N0:12, LCDR2 comprises SEQ ID N0:13, and LCDR3 comprises SEQ ID N0:14.

10. An epithelial membrane protein 2 (EMP2) inhibitor for use according to claim 9, wherein the antibody is a diabody.

11. An epithelial membrane protein 2 (EMP2) inhibitor for use according to claim 7, wherein the EMP2 inhibitor is an EMP2 si-RNA.

12. An epithelial membrane protein 2 (EMP2) inhibitor for use according to claim 11, wherein the EMP2 si-RNA comprises a target sequence wherein the target sequence comprises SEQ ID N0:3, SEQ ID N0:4, SEQ ID N0:5, or SEQ ID N0:6.

13. An epithelial membrane protein 2 (EMP2) inhibitor for use according to claim 11, wherein the EMP2 si-RNA comprises a target sequence selected from the group consisting of: SEQ ID N0:3, SEQ ID N0:4, SEQ ID N0:5, and SEQ ID N0:6.

## Patentansprüche

1. Hemmer für Epithelmembranprotein 2 (EMP2) zur Verwendung in der Behandlung von feuchter altersbedingter Makuladegeneration (age related macular degeneration, AMD), wobei der EMP2-Hemmer ausgewählt ist aus einer Gruppe bestehend aus einem Anti-EMP2-Antikörper, einer EMP2-si-RNA und Kombinationen davon.

2. Hemmer für Epithelmembranprotein 2 (EMP2) zur Verwendung nach Anspruch 1, wobei der EMP2-Hemmer ein Anti-EMP2-Antikörper ist.

3. Hemmer für Epithelmembranprotein 2 (EMP2) zur Verwendung nach Anspruch 1, wobei der EMP2-Hemmer eine EMP2-si-RNA ist.

4. Hemmer für Epithelmembranprotein 2 (EMP2) zur Verwendung nach Anspruch 1, wobei der EMP2-Hemmer mindestens zwei Hemmer für Epithelmembranprotein 2 (EMP2) umfasst.

5. Hemmer für Epithelmembranprotein 2 (EMP2) zur Verwendung nach Anspruch 4, wobei einer der mindestens zwei EMP2-Hemmer ein Anti-EMP2-Antikörper ist, und wobei einer der mindestens zwei EMP2-Hemmer eine EMP2-si-RNA ist.

6. Hemmer für Epithelmembranprotein 2 (EMP2) zur Verwendung in der Behandlung einer Erkrankung des Auges, wobei:
die Erkrankung des Auges diabetische Retinopathie, korneale Neovaskularisation, choroidale Neovaskularisation, Zyklitis, Hippel-Lindau-Krankheit, Frühgeborenen-Retinopathie, Pterygium, Histoplasmose, Iris-Neovaskularisation, Makulaödem, Glaukom-assoziierte Neovaskularisation und Purtscher-Retinopathie, und
der EMP2-Hemmer ausgewählt ist aus einer Gruppe bestehend aus einem Anti-EMP2-Antikörper, einer EMP2-si-RNA und Kombinationen davon.

7. Hemmer für Epithelmembranprotein 2 (EMP2) zur Verwendung in der Reduzierung der Expression vaskulärem endothelialem Wachstumsfaktor (vascular endothelial growth factor, VEGF) im Auge eines Patienten, der an einer Erkrankung leidet, ausgewählt aus der Gruppe bestehend aus feuchter altersbedingter Makuladegeneration (age related macular degeneration, AMD), diabetischer Retinopathie, kornealer Neovaskularisation, choroidaler Neovaskularisation, Zyklitis, Hippel-Lindau-Krankheit, Frühgeborenen-Retinopathie, Pterygium, Histoplasmose, Iris-Neovaskularisation, Makulaödem, Glaukom-assoziierter Neovaskularisation und Purtscher-Retinopathie,
wobei der EMP2-Hemmer ausgewählt ist aus einer Gruppe bestehend aus einem Anti-EMP2-Antikörper, einer EMP2-si-RNA und Kombinationen davon.

8. Hemmer für Epithelmembranprotein 2 (EMP2) zur Verwendung nach Anspruch 7, wobei der EMP2-Hemmer ein Anti-EMP2-Antikörper ist.

9. Hemmer für Epithelmembranprotein 2 (EMP2) zur Verwendung nach den Ansprüchen 1, 2, 6, 7 oder 8, wobei der Anti-EMP2-Antikörper eine schwere Kette und eine leichte Kette umfasst, wobei die schwere Kette drei Komplementarität-bestimmende Regionen (complementarity determining regions, CDRs) umfasst: HCDR1, HCDR2 und HCDR3, und wobei die leichte kette drei CDRs umfasst: LCDR1, LCDR2 und LCDR3, wobei HCDR1 die SEQ ID NO: 8 umfasst, HCDR2 die SEQ ID NO: 9 umfasst und HCDR3 die SEQ ID NO: 10 umfasst, und wobei LCDR1 die SEQ ID NO: 12 umfasst, LCDR2 die SEQ ID NO: 13 umfasst und LCDR3 die SEQ ID NO: 14 umfasst.

10. Hemmer für Epithelmembranprotein 2 (EMP2) zur Verwendung nach Anspruch 9, wobei der Antikörper ein Diabody ist.

11. Hemmer für Epithelmembranprotein 2 (EMP2) zur Verwendung nach Anspruch 7, wobei der EMP2-Hemmer eine EMP2-si-RNA ist.

12. Hemmer für Epithelmembranprotein 2 (EMP2) zur Verwendung nach Anspruch 11, wobei die EMP2-si-RNA eine Zielsequenz umfasst, wobei die Zielsequenz SEQ ID NO: 3, SEQ ID NO: 4, SEQ ID NO: 5 oder SEQ ID NO: 6 umfasst.

13. Hemmer für Epithelmembranprotein 2 (EMP2) zur Verwendung nach Anspruch 11, wobei die EMP2-si-RNA eine Zielsequenz umfasst, ausgewählt aus der Gruppe bestehend aus: SEQ ID NO: 3, SEQ ID NO: 4, SEQ ID NO: 5 und SEQ ID NO: 6.

## Revendications

1. Inhibiteur de protéine membranaire épithéliale 2 (EMP2) destiné à être utilisé dans le traitement de la dégénérescence maculaire humide liée à l'âge (DMLA humide), ledit inhibiteur d'EMP2 étant choisi dans le groupe constitué par un anticorps anti-EMP2, un pARNi d'EMP2 et une combinaison de ceux-ci.

2. Inhibiteur de protéine membranaire épithéliale 2 (EMP2) destiné à être utilisé selon la revendication 1, ledit inhibiteur d'EMP2 étant un anticorps anti-EMP2.

3. Inhibiteur de protéine membranaire épithéliale 2 (EMP2) destiné à être utilisé selon la revendication 1, ledit inhibiteur d'EMP2 étant un pARNi d'EMP2.

4. Inhibiteur de protéine membranaire épithéliale 2 (EMP2) destiné à être utilisé selon la revendication 1, ledit inhibiteur d'EMP2 comprenant au moins deux inhibiteurs de protéine membranaire épithéliale 2 (EMP2).

5. Inhibiteur de protéine membranaire épithéliale 2 (EMP2) destiné à être utilisé selon la revendication 4, l'un desdits au moins deux inhibiteurs d'EMP2 étant un anticorps anti-EMP2 et l'un desdits au moins deux inhibiteurs d'EMP2 étant un pARNi d'EMP2.

6. Inhibiteur de protéine membranaire épithéliale 2 (EMP2) destiné à être utilisé dans le traitement d'une maladie des yeux :
ladite maladie des yeux étant la rétinopathie diabétique, la néovascularisation cornéenne, la néovascularisation choroïdienne, la cyclite, la maladie de von Hippel-Lindau, la rétinopathie des prématurés, le ptérygion, l'histoplasmose, la néovascularisation iridienne, un oedème maculaire, une néovascularisation associée à un glaucome, et la rétinopathie de Purtscher, et
ledit inhibiteur d'EMP2 étant choisi dans le groupe constitué par un anticorps anti-EMP2, un pARNi d'EMP2 et une combinaison de ceux-ci.

7. Inhibiteur de protéine membranaire épithéliale 2 (EMP2) destiné à être utilisé dans la réduction de l'expression du facteur de croissance endothéliale vasculaire (VEGF) dans les yeux d'un patient souffrant d'une maladie choisie dans le groupe constitué par la dégénérescence maculaire humide liée à l'âge (DMLA humide), la rétinopathie diabétique, la néovascularisation cornéenne, la néovascularisation choroïdienne, la cyclite, la maladie de von Hippel-Lindau, la rétinopathie des prématurés, le ptérygion, l'histoplasmose, la néovascularisation iridienne, un oedème maculaire, une néovascularisation associée à un glaucome, et la rétinopathie de Purtscher,
ledit inhibiteur d'EMP2 étant choisi dans le groupe constitué par un anticorps anti-EMP2, un pARNi d'EMP2 et une combinaison de ceux-ci.

8. Inhibiteur de protéine membranaire épithéliale 2 (EMP2) destiné à être utilisé selon la revendication 7, ledit inhibiteur d'EMP2 étant un anticorps anti-EMP2.

9. Inhibiteur de protéine membranaire épithéliale 2 (EMP2) destiné à être utilisé selon les revendications 1, 2, 6, 7 ou 8, ledit anticorps anti-EMP2 comprenant une chaîne lourde et une chaîne légère, ladite chaîne lourde comprenant trois régions de détermination de complémentarité (CDR) : HCDR1, HCDR2 et HCDR3, et ladite chaîne légère comprenant trois CDR : LCDR1, LCDR2 et LCDR3, HCDR1 comprenant la SEQ ID n° : 8, HCDR2 comprenant la SEQ ID n° : 9, et HCDR3 comprenant la SEQ ID n° : 10, et LCDR1 comprenant la SEQ ID n° : 12, LCDR2 comprenant la SEQ ID n° : 13, et LCDR3 comprenant la SEQ ID n° : 14.

10. Inhibiteur de protéine membranaire épithéliale 2 (EMP2) destiné à être utilisé selon la revendication 9, ledit anticorps étant un dianticorps.

11. Inhibiteur de protéine membranaire épithéliale 2 (EMP2) destiné à être utilisé selon la revendication 7, ledit inhibiteur d'EMP2 étant un pARNi d'EMP2.

12. Inhibiteur de protéine membranaire épithéliale 2 (EMP2) destiné à être utilisé selon la revendication 11, ledit pARNi d'EMP2 comprenant une séquence cible, ladite séquence cible comprenant la SEQ ID n° : 3, la SEQ ID n° : 4, la SEQ ID n° : 5 ou la SEQ ID n° : 6.

13. Inhibiteur de protéine membranaire épithéliale 2 (EMP2) destiné à être utilisé selon la revendication 11, ledit pARNi d'EMP2 comprenant une séquence cible choisie dans le groupe constitué par : la SEQ ID n° : 3, la SEQ ID n° : 4, la SEQ ID n° : 5 et la SEQ ID n° : 6.
